# EUROPEAN PATENT SPECIFICATION

(11) **EP 3 485 832 B1**
(45) Date of publication and mention of the grant of the patent: **24.03.2021**
(21) Application number: 18842276.0
(22) Date of filing: 25.07.2018
(51) Int. Cl.: A61B 17/76, A61B 17/72, A61B 17/86, A61B 17/74

(54) **BLADE STRUCTURE, HIP SCREW, AND FEMORAL FRACTURE TREATMENT APPARATUS**
KLINGENSTRUKTUR, HÜFTSCHRAUBE UND OBERSCHENKELBRUCHBEHANDLUNGSVORRICHTUNG
STRUCTURE DE LAME, VIS DE HANCHE ET APPAREIL DE TRAITEMENT DE FRACTURE FÉMORALE

(30) Priority: 03.08.2017 CN 201710656631
(43) Date of publication of application: 22.05.2019
(73) Proprietor: Double Medical Technology Inc., Xiamen, Fujian 361000 (CN)
(72) Inventor: LIN, Zhixiong, Xiamen Fujian 361000 (CN); LONG, Qiang, Xiamen Fujian 361000 (CN); JIANG, Daohai, Xiamen Fujian 361000 (CN); WANG, Meng, Xiamen Fujian 361000 (CN); FAN, Guixiong, Xiamen Fujian 361000 (CN); LIU, Chongbing, Xiamen Fujian 361000 (CN); ZENG, Da, Xiamen Fujian 361000 (CN)
(74) Representative: Gulde & Partner
(86) International application number: PCT/CN2018/097061
(87) International publication number: WO 2019/024742

(56) References cited:
- EP-A1- 3 391 841
- WO-A2-2011/002903
- CN-A- 107 280 749
- CN-A- 107 320 168
- CN-U- 201 624 765
- CN-U- 201 664 338
- CN-U- 201 701 288
- CN-U- 205 286 490
- DE-A1- 4 318 150
- DE-U1-202004 018 748
- KR-B1- 101 401 829
- US-A1- 2017 202 584

## Description

### FIELD OF THE INVENTION

The present invention relates to a medical device, and more particularly, to a blade structure, and a hip screw with the blade structure and a femur fracture treatment device.

### BACKGROUD OF THE INVENTION

In terms of medicine, femur fracture is a disease in which a femur of a human body is broken partially or entirely due to injury. The femur fracture is difficult to recover. The fracture is very painful and difficult in moving and needs a long time to recover. The treatment of femur fracture must follow the three basic principles of reduction, fixation, and functional exercise. The reduction is to relocate the fracture caused already-misplaced femur fracture portion to its normal position as close as possible to restore the support function of the femur. To hold the skeletal structure after corrective reduction in place, a femur fracture treatment device is usually used.

Femur fracture usually occurs in the femoral neck and trochanteric regions. Typically, the trochanteric and sub-trochanteric femur fractures are currently treated with an intramedullary nail having a lateral through hole for holding a bone fastener therein. The intramedullary nail is assembled in the medullary cavity of the femur to partially prevent the torsion of the fracture portion. A hip screw passes through the lateral through hole of the intramedullary nail, then the neck of the femur and finally enters the femur. The hip screw is generally rotatably inserted into the femur, and after installation, the intramedullary nail and the hip screw cannot rotate relative to each other. To this end, it is common to provide a positioning bolt in the intramedullary nail to fix the hip screw which passes through the lateral through hole to prevent relative movement or rotation between the intramedullary nail and the hip screw, or to make a hip screw of non-circular configuration to prevent rotation of itself, but these measures still fail to completely suppress the rotation, lateral movement and the like of the fracture portion. Further, subject to long-term external force in use, the positioning part is twisted and loosened, which easily causes rotation and axial movement of the intramedullary nail, resulting in delayed bone healing process. Germany patent No.: DE202004018748U1 is relevant to but different from the solution of current invention.

### SUMMARY OF THE INVENTION

Based on this, it is necessary to provide a femur fixation device to overcome the problem of structural instability of a prior art femur fixation device during long-term use.

A blade structure (10), includes a body having a centrally radially symmetrical cross section, the centrally radially symmetrical cross section including a plurality of centrally radially symmetrical sections, on each of the symmetrical section at least a cutting slot (100) and two blades (200) formed at corresponding top ends of two sides of the cutting slot (100) respectively being defined. The cross section of the body is a circular cross section; a cross section of the cutting slot (100) includes a pair of first circular arcs (110) each of which runs from the blade (200) to the bottom wall of the cutting slot (100) and of each of which a circle center is located in a space defined together by the cutting slot (100) and the pair of first circular arcs (110) in the same cutting slot (100); and two first circular arcs (110) of the pair of the first circular arcs (110) are connected together through a second circular arc (120) of which a circle center coincides with a circle center of the cross section of the body of the blade structure (10); and a plurality of circular holes (23) is defined in the blade (200) of the blade structure (10).

The number of the cutting slots (100) is three.

The cutting slot is a straight cutting slot, and a lower portion of the cutting slot is connected smoothly with an outer surface of the blade structure (10).

The cutting slot is a threaded cutting slot with a predefined lead angle.

Also provided is a hip screw, comprising: a blade portion (21) and a connecting portion (22), wherein the blade portion (21) comprises the blade structure (10) as mentioned above.

The hip screw (20) is provided with the through hole (24) running along its center line.

A plurality of circular holes (23) is defined in the blade (200) of the blade structure (10).

A plurality of circular holes (23) is also defined in a lower portion of the cutting slot of the blade structure (10), and the circular holes (23) communicate with the through hole (24).

The present invention also provides a femur fracture treatment device including any one of the hip screws as mention above.

The connecting portion (22) is provided with a positioning groove (25) in a surface of the hip screw (20), and a length direction of the positioning groove (25) is consistent with that of the hip screw (20).

A plane parallel to the positioning groove (25) has a predefined inclination angle relative to an end surface of a top end of the connecting portion (22).

A bottom surface of the positioning slot is arc-shaped.

Stable connection between the structural body and bone structure is realized using the blade structure of special design. The blades of the blade structure are located at the top ends of two sides of the cutting slot respectively. A projection of a top end of each blade is located on a bottom wall of the cutting slot. By this way, an opening defined between the two blades is narrower than an interior defined together by the two blades and bottom wall. The hip screw with this kind of blade structure can be tightly combined with the bone structure after being embedded into the bone structure, thus easily avoiding the problem of rotation and unstable location.. This makes the femur fracture treatment device with the hip screw with the blade structure more stable in its location during long term use, and ensures the normal healing of the bone.

### BRIEF DESCRIPTION OF THE DRAWINGS

Figure 1 illustrates a cross sectional view of a blade structure according to one embodiment of the invention;
Figure 2 illustrates a front view of a hip screw according to one embodiment of the invention;
Figure 3 illustrates a cross sectional view of a hip screw according to one embodiment of the invention; and
Figure 4 illustrates a front view of a hip screw according to another embodiment of the invention.

### DETAILED DESCRIPTION OF THE INVENTION

Various exemplary embodiments of the present invention are further described below in conjunction with the accompanied drawings, wherein the same reference numerals refer to the same parts throughout the drawings. Further, if a detailed description of a known technique is unnecessary for showing the features of the present invention, it will be omitted.

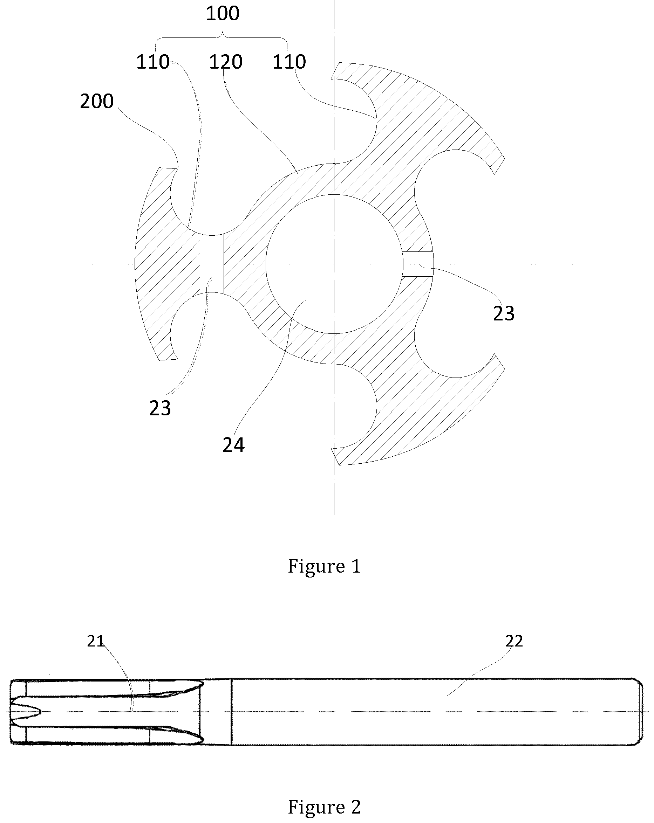

The present invention provides a blade structure, and as shown in figure 1, the blade structure 10 has a body of which a cross section is centrally radially symmetrical. The cross section may have a shape of circular, square, equilateral triangle, oval, rectangle and so on. The blade structure 10 has a plurality of centrally radially symmetrical sections. Defined on each of the sections are at least a cutting slot 100 and two blades 200 formed at corresponding top ends of two sides of the cutting slot 100 respectively. That is to say, the blades 200 are located at two sides of the cutting slot 100 respectively, and formed at corresponding top ends of two sides of the cutting slot 100 respectively. The projection of a top end of each blade 200 should be located on a bottom wall of the interior of cutting slot. By this way, an opening defined between the two blades is narrower than an interior defined by the two blades and bottom wall. The blade structure 10 of the present invention is of an embedded cutter structure. The cutter structure is applied to the hip screw, and is able to meet the working requirement of the hip screw. In an ideal state the hip screw should be embedded in an object and kept stationary, at least in the radial direction of the blade structure 10. That is to say, the hip screw will not be rotated around an axis when external force applies thereto, and the blade structure has a small opening and a relatively large internal cavity, so that after the blade structure 10 is embedded in the object, the cutting slot 100 is filled with much material of the object, which can generate great resistance to the rotation of the cutter structure, and avoid the radial positional change easily.

There are many structures of the small opening and a relatively large internal cavity, and as a preferred embodiment, the body of the blade structure 10 has a circular cross section. The cross section contour of the cutting slot 100 is not a straight contour, meaning that the cross section contour is not completely constructed of straight edges which are angled relative to each other. In detail, a cross section of the blade structure 10 includes a pair of first circular arcs 110 each of which runs from the blade 200 to the bottom wall of the cutting slot and of each of which a circle center is located in a space defined together by the cutting slot 100, and the pair of first circular arcs 110 in the same cutting slot 100; and a second circular arc 120 of which a circle center coincides with a circle center of the cross section of a body of the blade structure 10 and which is connected to the pair of the first circular arcs 110. The cross section of the cutting slot 100 is constructed of said second circular arc 120 and the pair of the first circular arcs 110 that are connected with the second circular arc 120; that is to say, the cross section of the cutting slot 100 is constructed of the pair of the first circular arcs 110 and said second circular arc 120. The second circular arc 120 is connected to the two first circular arcs 110. The circle centers of the two first circular arcs 110 are at the same side of a line, and the circle center of the second circle arc 120 is at the other side of the line. The circle center of the second circle arc 120 coincides with the circle center of the cross section of the blade structure 10, and the radii of the two arcs are different from each other. Because the blade structure 10 has a circular contour, the first circular arcs 110 and the circular contour of the blade structure 10 can intersect directly, and an arc tip orientated towards the interior of the circular contour of the blade structure 10 is formed at the intersecting portion (alternatively intersection is unnecessary here and in this case, an arc tip may be constructed by defining an opening inside of the first circular arcs) . As a result, the blade structure 10 forms a structure with a small opening and a relatively large internal cavity. The cross sectional arrangement of this kind can provide as many as possible hollow areas inside the cross section. The larger the hollow areas in the cross section are, the less interference resulted from the hip screw will be imposed on the intraosseous substance.

The hip screw 20 of the present invention, as shown in figures 1 and 2, includes a blade portion 21 and a connecting portion 22, wherein the blade portion 21 adopts the blade structure 10 as mentioned above. The hip screw 20 is used for being inserted into the human bones, with a result that the hip screw 20 has the characteristic of stabilization and with less or even no rotation after being embedded into the bone, which makes sure that the healing of bone is less influenced by external factors. As a preferred embodiment, the number of the cutting slots 100 distributed on the blade structure 10 is three, and in detail, there could be three cutting slots 100 of identical shape and distributed uniformly along the center of the blade structure 10. The design as mentioned above can make the area of the internal cavity of each cutting slot 100 as large as possible, and can also make the amount of material of the blade structure 10 as less as possible.

As a preferred embodiment, the cutting slot 100 is a straight cutting slot, and a lower portion of the cutting slot 100 is connected smoothly with an outer surface of the blade structure 10. The blade structure 10with the straight cutting slot 100 can be easily embedded into an object along a straight line direction because of special shape of the cross section. As a result, as shown in Figure 2, the hip screw 20 using the blade structure 10 of the straight cutting slot 100 has characteristics of easy implantation into bones and post-implantation stability.

As another preferred embodiment, the cutting slot 100 is threaded cutting slot 100 with a predefined lead angle (not shown). If a threaded cutting slot 100 is adopted, the stability will be high when the cutting slot 100 is embedded into an object, and the cutting slot will not be easily loosened along a radial direction or an axial direction. Further, a threaded structure facilitates axial movement. As a result, a cutter with a threaded cutting slot can avoid using additional tool, and can be embedded into an object by rotation of itself.

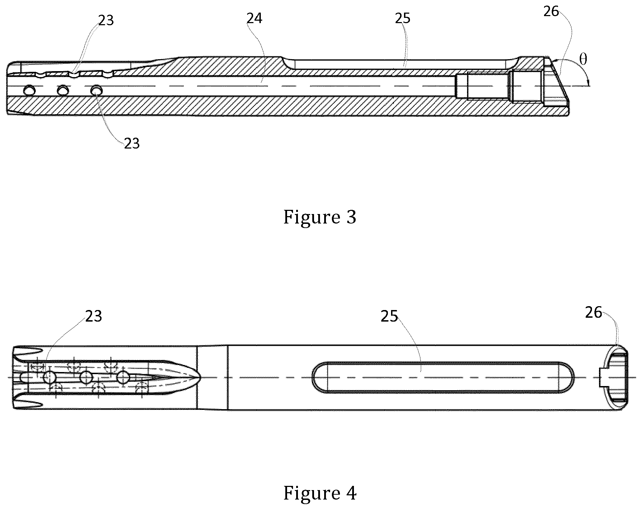

In solution of the hip screw 20 of the present invention, preferably, as shown in Figures 1 and 3, a through hole 24 is defined in a center of the hip screw 20, that is to say, the hip screw 20 is provided with the through hole 24 running along its center line. A guide pin can pass through a central portion of the hip screw 20. The guide pin plays a guiding role in the process of implantation of the hip screw 20, and brings convenience for operation.

As a preferred embodiment, as shown in Figures 1 and 3, a plurality of circular holes 23 is defined in the blade 200 of the hip screw 20. On one hand, the circular holes 23 can reduce the weight of the hip screw 20 making it lighter while keeping the structure stable, and on the other hand, the intra-bone material separated by the blade 200 can be exchanged through the circular holes 23, thereby reducing disturbance of the device to substances in the bone.

As a further preferred embodiment, as shown in Figures 1 and 4, a plurality of circular holes 23 is also defined in a lower portion of the cutting slot 100 of the blade structure 10 of the hip screw 20. The circular holes 23 communicate with the through hole 24 in the center of the hip screw 20. The circular holes 23 serve as cement holes, which are mainly used in case of osteoporosis in the elderly, and through which the bone cement is injected into the femur to enhance the stability of the hip screw 20.

Accordingly, a femur treatment device of the present invention includes an intramedullary nail and a hip screw, and cooperative structure of the two components is well known by person of ordinary skill in the art. The hip screw of the femur treatment device of the present invention is the hip screw 20 as mentioned above. And in an embodiment, a connecting portion 22 of the hip screw 20 is provided with a positioning groove 25, which is disposed in a surface of the hip screw 20, and a length direction of the positioning groove 25 is consistent with that of the hip screw 20. Generally, the hip screw 20 passes through a lateral hole in the intramedullary nail and cooperates with the same to form main structure of the femur treatment device. Therefore, the intramedullary nail is provided with a plug matched with the positioning groove 25. The plug is pressed against the positioning groove 25 to achieve an effect of fixing the position of the hip screw 20 relative to the intramedullary nail. Description of specific structure of the intramedullary nail is omitted herefrom.

As the positioning groove 25 is a groove defined in a curved surface, there must be a plane defined together by a length direction and width direction of the positioning groove 25 and parallel to the positioning groove 25. As a preferred embodiment, the plane has a predefined inclination angle θ with an end surface 26 of a top end of the connecting portion 22. That is to say, when the hip screw 20 is in the assembled position, the end surface 26 can be nearly parallel or completely parallel to the axis of the intramedullary nail. Such an arrangement makes the femur fracture treatment device more closely match the natural bone structure of the human body, and also makes the femur fracture treatment device more compact. In addition, the bottom surface of the positioning groove 25 may be provided in various shapes, for example, it may has a rectangular shape, a triangular shape or a circular arc shape, and preferably has a circular arc shape, as the circular arc-shaped bottom surface makes the contact area between a plug body provided on the intramedullary nail and the positioning groove 25 larger, so that the connection structure of the two is more stable. More preferably, suitably rounded corners are provided on each edge of the positioning slot 25 to make it easier to engage a mating structure such as a plug.

## Claims

1. A blade structure (10) for treating a femur fracture, comprising a body having a centrally radially symmetrical cross section, the centrally radially symmetrical cross section including a plurality of centrally radially symmetrical sections, on each centrally radially symmetrical section at least a cutting slot (100) and two blades (200) formed at corresponding top ends of two sides of the cutting slot (100) respectively being defined, wherein, the cross section of the body is a circular cross section; **characterised in that** a cross section of the cutting slot (100) includes a pair of first circular arcs (110) each of which runs from the blade (200) to the bottom wall of the cutting slot (100) and of each of which a circle center is located in a space defined together by the cutting slot (100) and the pair of first circular arcs (110) in the same cutting slot (100); and the pair of first circular arcs (110) are connected together through a second circular arc (120) of which a circle center coincides with a circle center of the cross section of the body of the blade structure (10); and a plurality of circular holes (23) is defined in one of the blades (200) of the blade structure (10).

2. The blade structure as recited in claim 1, wherein the number of the cutting slots (100) is three.

3. The blade structure as recited in claim 1, wherein the cutting slot is a straight cutting slot, and a lower portion of the cutting slot is connected smoothly with an outer surface of the blade structure (10).

4. The blade structure as recited in claim 1, wherein the cutting slot is a threaded cutting slot with a predefined lead angle.

5. A hip screw, comprising: a blade portion (21) and a connecting portion (22), wherein the blade portion (21) comprises the blade structure (10) as recited in any one of claims 1-4.

6. The hip screw as recited in claim 5, wherein the hip screw (20) is provided with the through hole (24) running along its center line.

7. The hip screw as recited in claim 6, wherein a plurality of circular holes (23) is defined in the blade (200) of the blade structure (10).

8. The hip screw as recited in claim 7, wherein a plurality of circular holes (23) is also defined in a lower portion of the cutting slot of the blade structure (10), and the circular holes (23) communicate with the through hole (24).

9. A femur fracture treatment device, comprising: the hip screw (20) as recited in any one of claims 5-8.

10. The femur fracture treatment device as recited in claim 9, wherein the connecting portion (22) is provided with a positioning groove (25) in a surface of the hip screw (20), and a length direction of the positioning groove (25) is consistent with that of the hip screw (20).

11. The femur fracture treatment device as recited in claim 10, wherein a plane parallel to the positioning groove (25) has a predefined inclination angle relative to an end surface of a top end of the connecting portion (22).

12. The femur fracture treatment device as recited in claim 10, wherein a bottom surface of the positioning slot is arc-shaped.

## Patentansprüche

1. Klingenstruktur (10) zur Behandlung eines Oberschenkelbruchs,
umfassend einen Körper mit einem zentralen radialen symmetrischen Querschnitt, wobei der zentrale radiale symmetrische Querschnitt eine Mehrzahl von zentralen radialen symmetrischen Bereichen umfasst, auf
jedem zentralen radialen symmetrischen Bereich mindestens ein Schneidschlitz (100) und zwei Klingen (200) an jeweils definierten entsprechenden oberen Enden von zwei Seiten des Schneidschlitzes (100) ausgebildet sind, wobei der Querschnitt des Körpers ein kreisförmiger Querschnitt ist;
**dadurch gekennzeichnet, dass**
ein Querschnitt des Schneidschlitzes (100) ein Paar erster kreisförmiger Bögen (110) umfasst, von denen jeder von der Klinge (200) zu der unteren Wand des Schneidschlitzes (100) läuft und von denen sich jeweils eine Kreismitte in einem Raum befindet, der von dem Schneidschlitz (100) und dem Paar erster kreisförmiger Bögen (110) zusammen in dem gleichen Schneidschlitz (100) definiert ist; und
das Paar erster kreisförmiger Bögen (110) durch einen zweiten kreisförmigen Bogen (120) miteinander verbunden ist, von dem eine Kreismitte mit einer Kreismitte des Querschnitts des Körpers der Klingenstruktur (10) übereinstimmt; und eine Mehrzahl von kreisförmigen Löchern (23) in einer der Klingen (200) der Klingenstruktur (10) definiert sind.

2. Klingenstruktur nach Anspruch 1, wobei die Anzahl der Schneidschlitze (100) drei ist.

3. Klingenstruktur (10) nach Anspruch 1, wobei der Schneidschlitz ein gerader Schneidschlitz ist und ein unterer Abschnitt des Schneidschlitzes ebenmäßig mit einer äußeren Oberfläche der Klingenstruktur verbunden ist.

4. Klingenstruktur nach Anspruch 1, wobei der Schneidschlitz ein Gewindeschneidschlitz mit einem vorbestimmten Anstellwinkel ist.

5. Hüftschraube, umfassend: einen Klingenabschnitt (21) und einen Verbindungsabschnitt (22), wobei der Klingenabschnitt (21) die Klingenstruktur (10) nach einem der Ansprüche 1-4 umfasst.

6. Hüftschraube nach Anspruch 5, wobei die Hüftschraube (20) so vorgesehen ist, dass das Durchgangsloch (24) entlang deren Mittellinie verläuft.

7. Hüftschraube nach Anspruch 6, wobei eine Mehrzahl von kreisförmigen Löchern (23) in der Klinge (200) der Klingenstruktur (10) definiert ist.

8. Hüftschraube nach Anspruch 7, wobei eine Mehrzahl von kreisförmigen Löchern (23) auch in einem unteren Abschnitt des Schneidschlitzes der Klingenstruktur (10) definiert ist und die kreisförmigen Löcher (23) in Verbindung mit dem Durchgangsloch (24) stehen.

9. Oberschenkelbruchbehandlungsvorrichtung, umfassend: die Hüftschraube (20) nach einem der Ansprüche 5-8.

10. Oberschenkelbruchbehandlungsvorrichtung nach Anspruch 9, wobei der Verbindungsabschnitt (22) mit einer Positionierungsnut (25) in einer Oberfläche der Hüftschraube (20) vorgesehen ist und eine Längenrichtung der Positionierungsnut (25) der der Hüftschraube (20) entspricht.

11. Oberschenkelbruchbehandlungsvorrichtung nach Anspruch 10, wobei eine Ebene parallel zu der Positionierungsnut (25) einen vorbestimmten Neigungswinkel relativ zu einer Endoberfläche eines oberen Endes des Verbindungsabschnittes (22) aufweist.

12. Oberschenkelbruchbehandlungsvorrichtung nach Anspruch 10, wobei eine untere Oberfläche des Positionierungsschlitzes bogenförmig ist.

## Revendications

1. Structure de lame (10) pour le traitement d'une fracture fémorale,
comprenant un corps ayant une section transversale à symétrie radiale centrale, la section transversale à symétrie radiale centrale comprenant une pluralité de sections transversales à symétrie radiale centrale, au moins une rainure de coupe (100) et deux lames (200) formées à des extrémités supérieures de deux côtés de la rainure de coupe (100) correspondantes sur chaque section à symétrie radiale centrale étant définies respectivement, la section transversale du corps étant une section transversale circulaire ;
**caractérisée en ce qu'**
une section transversale de la rainure de coupe (100) comprend une paire de premiers arcs circulaires (110), chacun desquels s'étendant de la lame (200) à la paroi inférieure de la rainure de coupe (100) et un centre de cercle de chacun desquels étant situé dans un espace défini conjointement par la la rainure de coupe (100) et la paire des premiers arcs circulaires (110) dans la même rainure de coupe (100) ; et
la paire des premiers arcs circulaires (110) étant reliés ensemble par un deuxième arc circulaire (120), un centre de cercle duquel coïncidant avec un centre de cercle de la section transversale du corps de la structure de lame (10) ; et une pluralité d'orifices circulaires (23) étant définis dans l'une des lames (200) de la structure de lame (10).

2. Structure de lame selon la revendication 1, le nombre de rainures de coupe (100) étant trois.

3. Structure de lame selon la revendication 1, la rainure de coupe étant une rainure de coupe droite, et une partie inférieure de la rainure de coupe étant reliée de façon lisse avec une surface extérieure de la structure de lame (10).

4. Structure de lame selon la revendication 1, la rainure de coupe étant une rainure de coupe filetée avec un angle de filet prédéfini.

5. Vis de hanche, comprenant : une partie de lame (21) et une partie de liaison (22), la partie de lame (21) comprenant la structure de lame (10) selon l'une quelconque des revendications 1-4.

6. Vis de hanche selon la revendication 5, la vis de hanche (20) étant fournie avec un orifice traversant (24) s'étendant dans la direction de sa ligne centrale.

7. Vis de hanche selon la revendication 6, une pluralité d'orifices circulaires (23) étant définis dans la lame (200) de la structure de lame (10).

8. Vis de hanche selon la revendication 7, une pluralité d'orifices circulaires (23) étant également définis dans une partie inférieure de la rainure de coupe de la structure de lame (10), et les orifices circulaires (23) communiquant avec l'orifice traversant (24).

9. Dispositif de traitement de fracture fémorale, comprenant : la vis de hanche (20) selon l'une quelconque des revendications précédentes 5-8.

10. Dispositif de traitement de fracture fémorale selon la revendication 9, la partie de liaison (22) étant fournie avec une rainure de positionnement (25) dans une surface de la vis de hanche (20), et une direction longitudinale de la rainure de positionnement (25) concordant avec celle de la vis de hanche (20).

11. Dispositif de traitement de fracture fémorale selon la revendication 10, un plan parallèle à la rainure de positionnement (25) ayant un angle d'inclinaison prédéfini par rapport à une surface d'extrémité d'une extrémité supérieure de la partie de liaison (22).

12. Dispositif de traitement de fracture fémorale selon la revendication 10, une surface inférieure de la rainure de positionnement étant en forme d'arc.
